# EUROPEAN PATENT SPECIFICATION

(11) **EP 3 834 764 B1**
(45) Date of publication and mention of the grant of the patent: **31.07.2024**
(21) Application number: 20213116.5
(22) Date of filing: 10.12.2020
(51) Int. Cl.: A61B 34/20, A61B 18/00, A61M 25/10, A61B 18/14, A61B 17/00, A61B 90/00, H05K 1/03, H05K 1/02, H05K 1/16, H05K 3/00

(54) **BALLOON CATHETER WITH POSITION SENSORS**
BALLONKATHETER MIT POSITIONSSENSOREN
CATHÉTER À BALLONNET DOTÉ DE CAPTEURS DE POSITION

(30) Priority: 11.12.2019 US 201916711344
(43) Date of publication of application: 16.06.2021
(73) Proprietor: Biosense Webster (Israel) Ltd., Yokneam 2066717 (IL)
(72) Inventor: GOVARI, Assaf, Yokneam 2066717 (IL)
(74) Representative: Carpmaels & Ransford LLP

(56) References cited:
- EP-A1- 3 381 396
- WO-A1-2020/104886
- US-A1- 2017 042 614
- US-A1- 2018 180 684
- US-A1- 2018 280 658
- US-A1- 2019 365 464

## Description

### FIELD OF THE INVENTION

The present invention relates generally to invasive medical probes, and specifically to balloon catheters.

### BACKGROUND

A balloon catheter comprises an inflatable balloon at its distal end that can be inflated and deflated as necessary. In operation, the balloon is typically deflated while the catheter is inserted into a body cavity (for example, a chamber of the heart) of a patient, and is then inflated within the cavity in order to perform the necessary procedure, and deflated again upon completing the procedure.

For example, U.S. Patent Application Publication 2018/0280658 describes a medical apparatus that includes a probe having a distal end configured for insertion into a body cavity and containing a lumen that opens through the distal end, and an inflatable balloon deployable through the lumen into the body cavity. The medical apparatus also includes a flexible printed circuit board having a first side attached to the exterior wall of the inflatable balloon and a second side opposite the first side, and an ultrasonic transducer mounted on the first side of the flexible printed circuit board and encapsulated between the exterior wall of the balloon and the flexible printed circuit board. In some embodiments, the medical apparatus may include an electrode mounted on the flexible circuit board and configured as a location sensor.

US 2018/0280658 A1 describes a balloon catheter for performing invasive procedures such as intracardiac ablation and anatomical mapping.

US 2018/0180684 A1 describes an apparatus, including a flexible insulating substrate.

### SUMMARY

The invention is defined by the appended claims. T Embodiments of the present invention that are described hereinbelow provide an improved apparatus for finding the position of a balloon catheter inside the body.

There is therefore provided, in accordance with an embodiment of the invention, a medical apparatus, including a flexible insertion tube having a distal end configured for insertion into a cavity in a body of a living subject and containing a lumen passing through the insertion tube to the distal end. An inflatable balloon is deployable from the distal end of the insertion tube and configured to be inflated by passage of a fluid through the lumen while the probe is deployed in the cavity in the body. At least one flexible circuit substrate is attached to a surface of the inflatable balloon. One or more electrodes, which include a conductive material, are disposed on an outer side of the at least one flexible circuit substrate so as to contact tissue in the cavity in the body when the balloon is inflated. A spiral conductive trace is disposed on the at least one flexible circuit substrate.

In some embodiments, the insertion tube has a proximal end configured for connection to a console, and the apparatus includes electrical wiring coupling the one or more electrodes and the spiral conductive trace to the console. In one embodiment, the apparatus includes signal generation circuitry, which is configured to supply electrical signals via the electrical wiring to the one or more electrodes so as to apply a therapeutic procedure to the tissue with which the one or more electrodes are in contact.

Additionally or alternatively, the apparatus includes position sensing circuitry, which is configured to receive, via the electrical wiring, signals that are output by the spiral conductive trace in response to a magnetic field that is applied to the body and to process the signals so as to derive position coordinates of the inflated balloon in the body. In a disclosed embodiment, the magnetic field includes multiple magnetic field components directed along different, respective axes, and the position sensing circuitry is configured to process the signals responsively to the multiple magnetic field components so as to derive both location and orientation coordinates of the inflated balloon in the body. Additionally or alternatively, the apparatus includes one or more magnetic field generators, which are configured to be positioned in proximity to the body and to apply the magnetic field thereto.

In some embodiments, the at least one flexible printed circuit substrate includes a plurality of flexible circuit substrates, which are distributed circumferentially around the inflatable balloon, and the one or more electrodes include multiple electrodes disposed respectively on the plurality of the flexible printed circuit substrates. In one such embodiment, the spiral conductive trace includes two or more spiral conductive traces disposed respectively on two or more of the flexible circuit substrates. The apparatus may additionally include position sensing circuitry, which is configured to receive respective signals that are output by the two or more spiral conductive traces in response to a magnetic field that is applied to the body, and to process the respective signals in combination so as to derive position coordinates of the inflated balloon in the body.

In a disclosed embodiment, the distal end of the flexible insertion tube is configured for insertion into a chamber of a heart of the subject.

There is also provided a method for position sensing, which includes providing a flexible insertion tube having a distal end configured for insertion into a cavity in a body of a living subject and containing a lumen passing through the insertion tube to the distal end. An inflatable balloon is coupled to be deployed from the distal end of the insertion tube and inflated by passage of a fluid through the lumen while the probe is deployed in the cavity in the body. At least one flexible printed circuit substrate is attached to a surface of the inflatable balloon. A conductive material is deposited on the at least one flexible circuit substrate so as to form one or more electrodes on an outer side of the flexible circuit substrate, whereby the one or more electrodes contact tissue in the cavity in the body when the balloon is inflated, and to form a spiral conductive trace on the at least one flexible circuit substrate.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for electrophysiological measurement and treatment in the heart, in accordance with an embodiment of the present invention; and
Fig. 2 is a schematic side view of the distal end of a balloon catheter deployed in a chamber of the heart, in accordance with an embodiment of the invention.

### DETAILED DESCRIPTION OF EMBODIMENTS

Balloon catheters are widely used in invasive therapeutic and diagnostic procedures, particularly inside chambers of the heart. Various methods are known in the art for finding the coordinates of the catheter and the balloon at its distal end within the heart. A magnetic sensor in the distal end of the catheter may be used to find the location and orientation coordinates of the catheter itself, and thus of the proximal end of the balloon, which is attached to the catheter. This sort of measurement is generally not sufficient, however, to give an accurate indication of the coordinates of the distal side of the balloon and of the electrodes that are disposed around the outer surface of the balloon, because the shape and size of the balloon change substantially as a function of inflation pressure within the balloon and of contact pressure between the outer surface of the balloon and the tissue in the heart.

In some balloon catheterization systems, such as the system described in the above-mentioned U.S. Patent Application Publication 2018/0280658, the location of the balloon is estimated by measuring the impedance between an electrode on the balloon and electrodes on the body surface. Such methods, however, are inaccurate, and enable the system to estimate only the location coordinates of the balloon, and not the orientation.

In response to this deficiency in systems that are known in the art, embodiments of the present invention provide a balloon catheter with additional magnetic position sensors, in the form of one or more spiral conductive traces on the surface of the balloon. (The term "spiral," as used in the present description and in the claims, refers to a path that winds around a central point, with each successive turn of the path approaching or receding from the central point, depending on the direction in which the path is traversed. The turns of the spiral may be curved or rectangular or have any other suitable shape.) Each such spiral trace acts as a coil, and outputs an electrical signal when placed in a magnetic field. The electrical signals from these coils can be processed to find both the location and orientation of the entire balloon, including the distal side of the balloon, regardless of variations in the size and shape of the balloon due to internal and external pressures.

In the disclosed embodiments, medical apparatus comprises a flexible insertion tube configured for insertion into a cavity in a body of a living subject, such as a chamber of the heart. An inflatable balloon is deployed from the distal end of the insertion tube, with at least one flexible circuit substrate attached to the surface of the balloon. One or more electrodes, which comprise a conductive material, are deposited or otherwise disposed on the outer side of the flexible circuit substrate, along with a spiral conductive trace, which serves as a coil. In some embodiments, multiple flexible circuit substrates are distributed circumferentially around the balloon, with electrodes and spiral conductive traces formed one some or all of the circuit substrates. Once the distal end of the insertion tube is in place in the cavity in the body, the balloon is inflated by passage of a fluid through a lumen in the insertion tube, and thus contacts tissue in the cavity in the body.

To find the position (location and orientation) coordinates of the inflated balloon, a magnetic field is applied to the body. Position sensing circuitry receives and processes the signals output by the spiral conductive traces in order to derive the position coordinates. Because of space and size constraints, the coils formed by the spiral conductive traces generally have small diameter (for example, about 2 mm) and relatively few turns, and therefore may output only weak signals. When spiral conductive traces are formed on multiple flexible circuit substrates, the respective signals can be processed in combination in order to derive position coordinates with improved signal/noise ratio and thus enhanced accuracy.

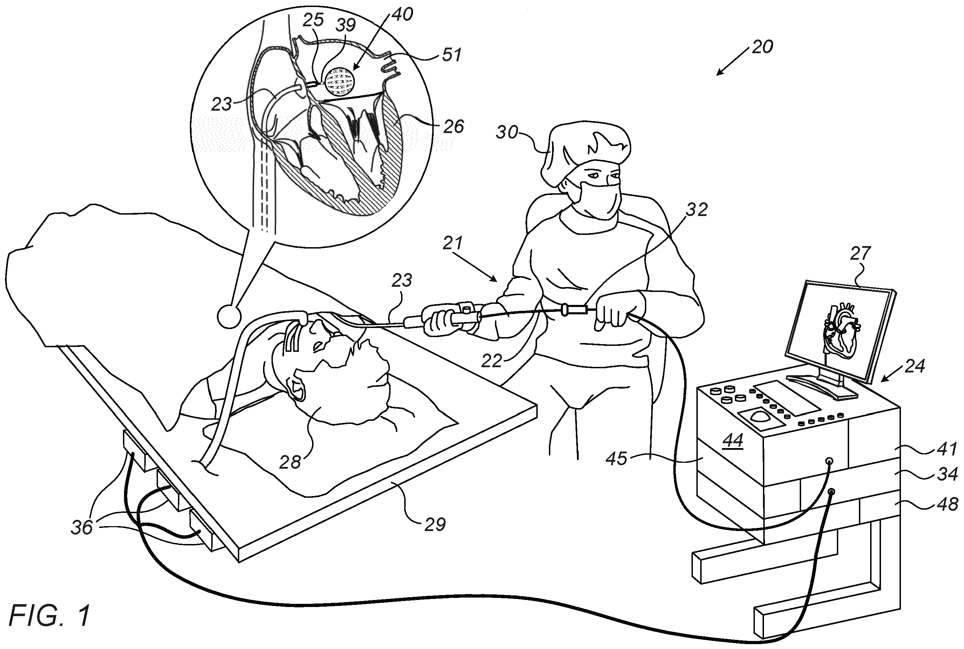

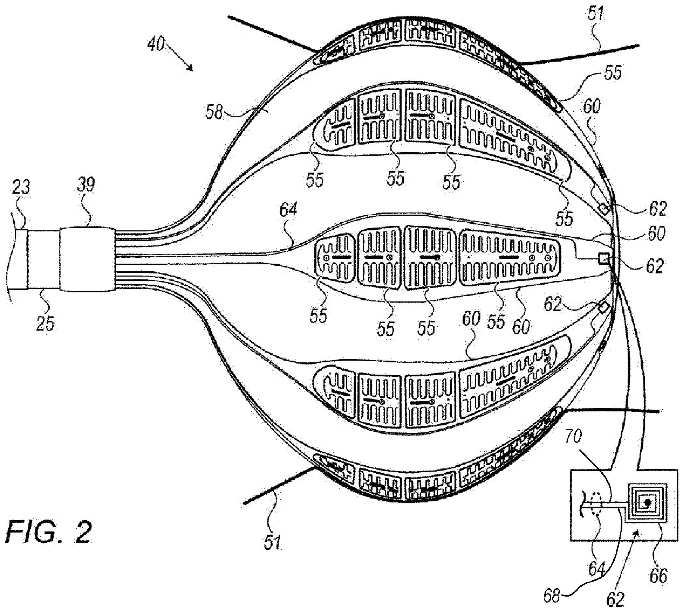

Fig. 1 is a schematic, pictorial illustration of a catheter-based system 20 for electrophysiological (EP) sensing and treatment of the heart, in accordance with an embodiment of the present invention. System 20 comprises a catheter 21, comprising an insertion tube 22 for transvascular insertion into a heart 26 of a patient 28, who is shown lying on a table 29. An inflatable balloon 40 is deployed at a distal end 25 of insertion tube 22 (as seen in the inset in Fig. 1). In the pictured embodiment, balloon 40 is applied in a therapeutic procedure, such as ablating tissue around an ostium 51 of a pulmonary vein in the left atrium of heart 26. Details of the structure and functionality of balloon 40 are described below with reference to Fig. 2.

The proximal end of catheter 21 is connected to a control console 24 comprising a power source 45, which typically includes radio-frequency (RF) signal generation circuitry. Power source 45 supplies RF electrical signals via electrical wiring running through insertion tube 22 to electrodes on balloon 40 so as to apply a therapeutic procedure to the tissue with which the electrodes are in contact. For example, depending on the voltage, frequency and power of the RF electrical signals, balloon 40 may be applied in treating arrhythmias in heart 26 by RF ablation or by irreversible electroporation (IRE) of the heart tissue. Additionally or alternatively, electrodes on balloon may be used in EP sensing and mapping of electrical signals in heart 26.

To carry out a therapeutic or diagnostic procedure, a physician 30 first inserts a sheath 23 into heart 26 of patient 28, and then passes insertion tube 22 through the sheath. Physician 30 advances distal end 25 of insertion tube 22 toward a target location in heart 26, for example in proximity to ostium 51, by manipulating catheter 21 using a manipulator 32 near the proximal end of the catheter. During the insertion of insertion tube 22, balloon 40 is deflated and is maintained in a collapsed configuration by sheath 23.

Once distal end 25 of insertion tube 22 has reached the left atrium in heart 26, physician 30 retracts sheath 23, partially inflates balloon 40, and further manipulates catheter 21 so as to navigate the balloon to the target location within ostium 51 of the pulmonary vein. When balloon 40 has reached the target location, physician 30 fully inflates balloon 40, so that electrodes disposed circumferentially around the balloon (Fig. 2) contact tissue around the ostium. Console 24 may verify that the electrodes are in good contact with the tissue by measuring the impedance between each of the electrodes and the tissue. Once good contact has been established, physician 30 actuates power source 45 to apply RF power to the tissue.

During this procedure, system 20 applies magnetic position sensing in tracking the location and orientation of insertion tube 22 and balloon 40 within heart 26, and thus guides physician 30 in maneuvering the balloon to the target location (within ostium 51 in the present example) and verifying that the balloon is properly in place. For this purpose, as shown in the inset in Fig. 1, distal end 25 of insertion tube 22 contains a magnetic position sensor 39, in a location slightly proximal to balloon 40. One or more magnetic field generators 36 are fixed in known positions in proximity to the body of patient 28, for example under bed 29 as shown in Fig. 1. A driver circuit 34 in console 24 applies drive signals to the magnetic field generators so as to produce multiple magnetic field components directed along different, respective axes. During navigation of distal end 25 in heart 26, magnetic sensor 39 outputs signals in response to the magnetic field components. Position sensing circuitry, such as a processor 41 in console 24, receives these signals via interface circuits 44, and processes the signals in order to find the location and orientation coordinates of distal end 25. These coordinates also indicate the location and orientation of the proximal end of balloon 40, which is deployed from distal end 25 of insertion tube 22.

In addition, as shown in Fig. 2, balloon 40 itself has one or more sensing coils on its surface, in the form of spiral conductive traces. These sensing coils likewise output signals in response to the magnetic fields applied by magnetic field generators 36. Processor 41 processes these signals in order to derive location and orientation coordinates of the inflated balloon, and specifically of the distal part of the balloon, which contacts the tissue in heart 26. Processor 41 presents the coordinates of balloon 40 on a display 27, for example by superimposing a graphical representation of the balloon, in the location and orientation indicated by the position sensors, on a three-dimensional map of the heart chamber in which the balloon is located.

The methods and apparatus for magnetic position sensing that are implemented in system 20 are based on those that are used in the CARTO^{®} system, produced by Biosense Webster, Inc. (Irvine, California). The principles of operation of this sort of magnetic sensing are described in detail, for example, in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all hereby referenced

Alternatively, system 20 may implement other magnetic position sensing technologies that are known in the art.

In some embodiments, processor 41 comprises a general-purpose computer, with suitable interface circuits 44 for receiving signals from catheter 21 (including low-noise amplifiers and analog/digital converters), as well as for receiving signals from and controlling the operation of the other components of system 20. Processor 41 typically performs these functions under the control of software stored in a memory 48 of system 20. The software may be downloaded to the computer in electronic form, over a network, for example, or it may, alternatively or additionally, be provided and/or stored on non-transitory tangible media, such as magnetic, optical, or electronic memory. Additionally or alternatively, at least some of the functions of processor 41 may be carried out by dedicated or programmable hardware logic.

Fig. 2 is a schematic side view of balloon 40, deployed from distal end 25 of insertion tube 22, in accordance with an embodiment of the invention. Balloon 40 is shown in this figure in its inflated state within ostium 51. Balloon 40 is typically inflated by passage of a fluid, such as saline solution, through a lumen (not shown) in insertion tube 22.

Balloon 40 is typically formed from a flexible biocompatible material such as polyethylene terephthalate (PET), polyurethane, nylon, or silicone. Multiple flexible circuit substrates 60 are attached to an outer surface 58 of balloon 40, for example using a suitable epoxy or other adhesive, and are distributed circumferentially around balloon 40. Substrates 60 comprise a suitable dielectric material, such as a polyimide, on which electrical traces can be deposited and etched using printed circuit fabrication techniques that are known in the art. Prior to attachment of substrate 60 to outer surface 58, electrodes 55 are formed on the outer sides of substrates 60 by depositing and etching a suitable conductive material, such as gold. Electrodes 55 will thus contact tissue in heart 26, such as the tissue of ostium 51, when balloon 40 is inflated.

Spiral conductive traces 66 are deposited on substrates 60 in a similar fashion to electrodes, and serve as magnetic sensing coils 62. The dimensions of sensing coils 62 are limited by the available space on substrates 60, for example to about 2 x 2 mm. For enhanced sensitivity, traces 66 typically have a fine pitch, for example 0.4 mm or less, and may be covered by an insulating coating to prevent short-circuiting of the traces by body tissue and fluids. Electrical wiring 64 couples sensing coils 62 through insertion tube 22 to console 24, and electrodes 55 are coupled by wiring to the console in similar fashion. (Conductive traces may be formed on both sides of substrate 60, or deposited in multiple layers on the substrate, using printed circuit fabrication techniques that are known in the art, to enable connection of wiring 64 to the central point of coils 62.) In the embodiment shown in Fig. 2, conductive trace 66 in the form of a rectilinear spiral is connected to or extend as part of trace 68 and trace 70 that extends through the catheter shaft 25 back to the handle so that coil 62 could be used to detect the magnetic field generators as referenced to the patient. Other variations of the coil 62 as well as methods are described and illustrated in Patent Application US20180180684, which is hereby referenced.

As explained above, processor 41 receives and processes the signals that are output by sensing coils 62 in response to the magnetic fields produced by magnetic field generators 36, and thus derives both location and orientation coordinates of the distal side of inflated balloon 40 in heart 26. In the pictured embodiment, sensing coils 62 are formed on multiple different substrates 60 at different locations around balloon 40. Processor 41 processes the respective signals that are output by sensing coils 62 on in combination, for example, by finding a directional average of the position coordinates of the multiple sensing coils. Processor 41 is thus able to derive position coordinates of the inflated balloon with enhanced accuracy.

Although the embodiments described above relate specifically to ablation therapies in the heart within and around the pulmonary veins, the principles of the present invention may similarly be applied, *mutatis mutandis,* in other therapeutic and diagnostic procedures within the heart, as well as in other body cavities. It will thus be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art, provided they are within the scope of the appended claims.

## Claims

1. Medical apparatus, comprising:
a flexible insertion tube (22) having a distal end (25) configured for insertion into a cavity in a body of a living subject (28) and containing a lumen passing through the insertion tube to the distal end;
an inflatable balloon (40) deployable from the distal end of the insertion tube and configured to be inflated by passage of a fluid through the lumen while the probe is deployed in the cavity in the body;
at least one flexible circuit substrate (60) attached to a surface (58) of the inflatable balloon; and
one or more electrodes (55), which comprise a conductive material disposed on an outer side of the at least one flexible circuit substrate so as to contact tissue in the cavity in the body when the balloon is inflated;
**characterised in that** the medical apparatus comprises further
a spiral conductive trace (66) disposed on the at least one flexible circuit substrate.

2. The apparatus according to claim 1, wherein the insertion tube has a proximal end configured for connection to a console (24), and the apparatus comprises electrical wiring (64) coupling the one or more electrodes and the spiral conductive trace to the console.

3. The apparatus according to claim 2, and comprising signal generation circuitry, which is configured to supply electrical signals via the electrical wiring to the one or more electrodes so as to apply a therapeutic procedure to the tissue with which the one or more electrodes are in contact.

4. The apparatus according to claim 2, and comprising position sensing circuitry (41), which is configured to receive, via the electrical wiring, signals that are output by the spiral conductive trace in response to a magnetic field that is applied to the body and to process the signals so as to derive position coordinates of the inflated balloon in the body.

5. The apparatus according to claim 4, wherein the magnetic field comprises multiple magnetic field components directed along different, respective axes, and wherein the position sensing circuitry is configured to process the signals responsively to the multiple magnetic field components so as to derive both location and orientation coordinates of the inflated balloon in the body.

6. The apparatus according to claim 4, and comprising one or more magnetic field generators (36), which are configured to be positioned in proximity to the body and to apply the magnetic field thereto.

7. The apparatus according to claim 1, wherein the at least one flexible circuit substrate comprises a plurality of flexible circuit substrates, which are distributed circumferentially around the inflatable balloon, and the one or more electrodes comprise multiple electrodes disposed respectively on the plurality of the flexible circuit substrates.

8. The apparatus according to claim 7, wherein the spiral conductive trace comprises two or more spiral conductive traces disposed respectively on two or more of the flexible circuit substrates.

9. The apparatus according to claim 8, and comprising position sensing circuitry, which is configured to receive respective signals that are output by the two or more spiral conductive traces in response to a magnetic field that is applied to the body, and to process the respective signals in combination so as to derive position coordinates of the inflated balloon in the body.

10. The apparatus according to claim 1, wherein the distal end of the flexible insertion tube is configured for insertion into a chamber of a heart of the subject (26).

## Patentansprüche

1. Medizinische Einrichtung, umfassend:
einen flexiblen Einführungsschlauch (22), der ein distales Ende (25) aufweist, der für eine Einführung in einen Hohlraum in einem Körper eines lebenden Subjekts (28) konfiguriert ist und ein Lumen enthält, das durch den Einführungsschlauch zu dem distalen Ende hindurchgeht;
einen aufblasbaren Ballon (40), der von dem distalen Ende des Einführungsschlauchs aus einsetzbar und konfiguriert ist, um durch einen Durchgang eines Fluids durch das Lumen aufgeblasen zu werden, während die Sonde in den Hohlraum in dem Körper eingesetzt ist;
mindestens ein flexibles Schaltungssubstrat (60), das an einer Oberfläche (58) des aufblasbaren Ballons befestigt ist; und
eine oder mehrere Elektroden (55), die ein leitfähiges Material umfassen, das auf einer Außenseite des mindestens einen flexiblen Schaltungssubstrats angeordnet sind, um, wenn der Ballon aufgeblasen ist, mit dem Gewebe in dem Hohlraum in dem Körper in Kontakt zu kommen;
**dadurch gekennzeichnet, dass** die medizinische Einrichtung ferner umfasst
eine spiralförmige leitende Spur (66), die auf dem mindestens einen flexiblen Schaltungssubstrat angeordnet ist.

2. Einrichtung nach Anspruch 1, wobei der Einführungsschlauch ein proximales Ende aufweist, das für eine Verbindung mit einer Konsole (24) konfiguriert ist, und die Einrichtung eine elektrische Verdrahtung (64) umfasst, die die eine oder die mehreren Elektroden und die spiralförmige leitende Spur mit der Konsole koppelt.

3. Einrichtung nach Anspruch 2 und umfassend einen Signalerzeugungsschaltkreis, der konfiguriert ist, um, über die elektrische Verdrahtung, elektrische Signale an die eine oder die mehreren Elektroden zu liefern, um ein therapeutisches Verfahren auf das Gewebe anzuwenden, mit dem die eine oder die mehreren Elektroden in Kontakt sind.

4. Einrichtung nach Anspruch 2 und umfassend einen Positionserfassungsschaltkreis (41), der konfiguriert ist, um, über die elektrische Verdrahtung, Signale zu empfangen, die durch die spiralförmige leitende Spur als Reaktion auf ein Magnetfeld, das an den Körper angelegt wird, ausgegeben werden, und um die Signale zu verarbeiten, um Positionskoordinaten des aufgeblasenen Ballons in dem Körper abzuleiten.

5. Einrichtung nach Anspruch 4, wobei das Magnetfeld mehrere Magnetfeldkomponenten umfasst, die entlang unterschiedlicher jeweiliger Achsen gerichtet sind, und wobei der Positionserfassungsschaltkreis konfiguriert ist, um die Signale als Reaktion auf die mehreren Magnetfeldkomponenten zu verarbeiten, um sowohl Standort- als auch Ausrichtungskoordinaten des aufgeblasenen Ballons in dem Körper abzuleiten.

6. Einrichtung nach Anspruch 4 und umfassend einen oder mehrere Magnetfeldgeneratoren (36), die konfiguriert sind, um in der Nähe des Körpers positioniert zu werden und um das Magnetfeld darauf anzulegen.

7. Einrichtung nach Anspruch 1, wobei das mindestens eine flexible Schaltungssubstrat eine Vielzahl von flexiblen Schaltungssubstraten umfasst, die kreisförmig um den aufblasbaren Ballon verteilt sind, und die eine oder die mehreren Elektroden mehrere Elektroden umfassen, die jeweils auf der Vielzahl der flexiblen Schaltungssubstrate angeordnet sind.

8. Einrichtung nach Anspruch 7, wobei die spiralförmige leitende Spur zwei oder mehr spiralförmige leitende Spuren umfasst, die jeweils auf zwei oder mehr der flexiblen Schaltungssubstrate angeordnet sind.

9. Einrichtung nach Anspruch 8 und umfassend einen Positionserfassungsschaltkreis, der konfiguriert ist, um jeweilige Signale zu empfangen, die durch die zwei oder mehr spiralförmigen leitenden Spuren als Reaktion auf ein Magnetfeld, das auf den Körper angelegt wird, auszugeben, und die jeweiligen Signale in Kombination zu verarbeiten, um Positionskoordinaten des aufgeblasenen Ballons in dem Körper abzuleiten.

10. Einrichtung nach Anspruch 1, wobei das distale Ende des flexiblen Einführungsschlauchs für die Einführung in eine Herzkammer des Subjekts (26) konfiguriert ist.

## Revendications

1. Appareil médical, comprenant :
un tube d'insertion flexible (22) ayant une extrémité distale (25) configurée pour être insérée dans une cavité du corps d'un sujet vivant (28) et contenant une lumière traversant le tube d'insertion jusqu'à l'extrémité distale ;
un ballon gonflable (40) déployable à partir de l'extrémité distale du tube d'insertion et configuré pour être gonflé par le passage d'un fluide dans la lumière lorsque la sonde est déployée dans la cavité du corps ;
au moins un substrat de circuit flexible (60) fixé à une surface (58) du ballon gonflable ; et
une ou plusieurs électrodes (55), constituées d'un matériau conducteur, disposées sur une face extérieure du substrat du circuit flexible au moins, de manière à entrer en contact avec les tissus de la cavité du corps lorsque le ballon est gonflé ;
**caractérisé en ce que** l'appareil médical comprend en outre
une trace conductrice en spirale (66) disposée sur l'au moins un substrat de circuit flexible.

2. Appareil selon la revendication 1, dans lequel le tube d'insertion a une extrémité proximale configurée pour être connectée à une console (24), et l'appareil comprend un câblage électrique (64) reliant l'une ou plusieurs électrodes et la trace conductrice en spirale à la console.

3. Appareil selon la revendication 2, comprenant un circuit de génération de signaux, configuré pour fournir des signaux électriques par l'intermédiaire du câblage électrique à une ou plusieurs électrodes afin d'appliquer une procédure thérapeutique au tissu avec lequel la ou les électrodes sont en contact.

4. Appareil selon la revendication 2, comprenant un circuit de détection de position (41), configuré pour recevoir, par l'intermédiaire du câblage électrique, des signaux émis par la trace conductrice en spirale en réponse à un champ magnétique appliqué au corps et pour traiter les signaux de manière à déduire les coordonnées de position du ballon gonflé dans le corps.

5. Appareil selon la revendication 4, dans lequel le champ magnétique comprend plusieurs composantes de champ magnétique dirigées le long de différents axes respectifs, et dans lequel le circuit de détection de position est configuré pour traiter les signaux en réponse aux multiples composantes de champ magnétique de manière à dériver les coordonnées de localisation et d'orientation du ballon gonflé dans le corps.

6. Appareil selon la revendication 4, et comprenant un ou plusieurs générateurs de champ magnétique (36), configurés pour être positionnés à proximité du corps et pour y appliquer le champ magnétique.

7. Appareil selon la revendication 1, dans lequel au moins un substrat de circuit flexible comprend une pluralité de substrats de circuit flexible, qui sont distribués circonférentiellement autour du ballon gonflable, et l'une ou plusieurs électrodes comprennent des électrodes multiples disposées respectivement sur la pluralité des substrats de circuit flexible.

8. Appareil selon la revendication 7, dans lequel la trace conductrice en spirale comprend deux traces conductrices en spirale ou plus, disposées respectivement sur deux substrats de circuit flexible ou plus.

9. Appareil selon la revendication 8, et comprenant un circuit de détection de position, configuré pour recevoir les signaux respectifs émis par les deux ou plusieurs traces conductrices en spirale en réponse à un champ magnétique appliqué au corps, et pour traiter les signaux respectifs en combinaison afin de déduire les coordonnées de position du ballon gonflé dans le corps.

10. Appareil selon la revendication 1, dans lequel l'extrémité distale du tube d'insertion flexible est configurée pour être insérée dans une chambre du coeur du sujet (26).
